# EUROPEAN PATENT APPLICATION

(11) **EP 2 789 339 A1**
(43) Date of publication of application: **15.10.2014**
(21) Application number: 14175944.9
(22) Date of filing: 12.03.2007
(51) Int. Cl.: A61K 31/5575, A61K 9/08, A61K 47/18, A61K 47/44, A61P 27/02, A61K 9/00

(54) **Aqueous composition comprising 15-keto-prostaglandin**

(30) Priority: 13.03.2006 JP 2006067764
(62) Divisional of application: 07738306.5
(71) Applicant: R-Tech Ueno, Ltd., Tokyo 100-0011 (JP); Sucampo AG, 6300 Zug (CH)
(72) Inventor: Hirata, Ryu, Chiyoda-ku, Tokyo 100-0011 (JP); Harada, Yasuhiro, Chiyoda-ku, Tokyo 100-0011 (JP); Ueno, Ryuji, Montgomery, MD Maryland 20854 (US)
(74) Representative: Beckmann, Claus

(57) **Abstract**

In an aqueous composition comprising a 15-keto-prostaglandin compound as an active ingredient, a polyoxyethylene castor oil derivative, which is polyoxyethylene 35 castor oil, is admixed. By admixing a polyoxyethylene castor oil derivative, the solubility of the 15-keto-PG compound in water is increased. In a preferable embodiment, the aqueous composition may further comprise a quaternary ammonium type cationic surface active agent as a preservative. The composition of this embodiment exhibits high antimicrobial preservative effectiveness against indigenous bacterium in the eyes such as *Staphylococcus aureus* and therefore, the composition is especially useful as ophthalmic composition for ocular topical administration.

## Description

### FIELD OF THE INVENTION

The present invention relates to an aqueous composition for topical administration, especially, for ophthalmic use, comprising a therapeutically effective 15-keto-prostaglandin compound as an active ingredient.

### BACKGROUND ART

Prostaglandins (hereinafter, referred to as PG(s)) are members of class of organic carboxylic acids, which are contained in tissues or organs of human and other mammals, and exhibit a wide range of physiological activities. PGs found in nature (primary PGs) have, as a general structural property thereof, a prostanoic acid skeleton as shown in the formula (A):

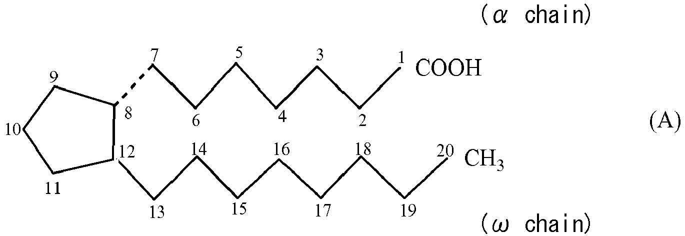

On the other hand, some synthetic PG analogues have modified skeletons. The primary PGs are classified into PGAs, PGBs, PGCs, PGDs, PGEs, PGFs, PGGs, PGHs, PGIs and PGJs on the basis of the structural property of the five membered ring moiety, and further classified into the following three types by the number and position of the unsaturated bond in the carbon chain moiety.
Type 1 (subscript 1): 13,14-unsaturated-15-OH
Type 2 (subscript 2): 5,6- and 13,14-diunsaturated-15-OH
Type 3 (subscript 3): 5,6-, 13,14-, and 17,18-triunsaturated-15-OH.

Further, PGFs are classified on the basis of the configuration of the hydroxy group at the 9-position into α type (wherein the hydroxy group is of the α-configuration) and β type (wherein the hydroxy group is of the β-configuration).

In addition, some 15-keto-PGs (PGs having an oxo group at position 15 in place of the hydroxy group) and 13,14-dihydro (having a single bond between positions 13 and 14)-15-keto-PGs have been known as substances naturally produced by enzymatic actions during metabolism of the primary PGs and have some therapeutic effect. 15-keto-PGs have been disclosed in USP Nos. 5,073,569, 5,534,547, 5,225,439, 5,166,174, 5,428,062 5,380,709 5,886,034 6,265,440, 5,106,869, 5,221,763, 5,591,887, 5,770,759 and 5,739,161, the contents of these references are herein incorporated by reference.

For example, 15-keto-prostaglandin compounds have been known to be useful for the treatment of glaucoma, see USPs 5,001,153 and 5,151,444, the contents of these references are herein incorporated by reference. Pharmaceutical composition for topical ophthalmic administration such as a composition for the treatment of glaucoma, in general, is preferably an aqueous formulation such as ophthalmic solution or eye drops. However, prostaglandin compounds in general are less soluble in water and therefore, a dissolving agent such as a surface active agent is added to an aqueous formulation comprising the PG compound.

Polyoxyethylene castor oil derivative, which is one of nonionic surface active agents, has been well known as a dissolving agent. An aqueous ophthalmic solution comprising a PG compound having hydroxy group at the 15-position and a polyoxyethylene castor oil derivative has been known. By adding polyoxyethylene castor oil derivative to the composition, the shelf stability of the 15-hydroxy-PG compound is improved (USP Nos. 5,849,792 and 6,011,062).

An ophthalmic solution comprising a difluoro PGF2α derivative having two fluorine atoms at the 15-position instead of the hydroxy group and a polyoxyethylene castor oil derivative has been known. It also is disclosed that by adding polyoxyethylene castor oil derivative to the composition, the adsorption of the difluoro PGF_{2α} derivative to the resin made containers can be suppressed (WO02/22131).

However, no composition comprising a 15-keto-PG compound, i.e. a PG compound having an oxo group instead of the hydroxy group at the 15-position and a polyoxyethylene castor oil derivative has been known. In addition, the art does not know the effect of the polyoxyethylene castor oil derivative when it is used as a dissolving agent.

On the other hand, quaternary ammonium type cationic surface active agents such as benzalkonium chloride and benzethonium chloride have been used as preservatives in pharmaceutical formulations. In the case of an active ingredient for an aqueous formulation is insoluble in water, a dissolving agent is usually added to the aqueous formulation. Accordingly, it is important for the drug formulation design to confirm how the dissolving agent affect the property of the preserving agent contained in the aqueous formulation.

Antimicrobial preservative effectiveness test is provided by the Japanese Pharmacopoeia. According to the protocol, specified species of microorganisms are inoculated to the composition to be tested and the time course of the growth of the respective microorganisms is observed to evaluate the preservation property of the composition.

*Staphylococcus aureus* is an indigenous bacterium in the eyes, especially in tunica conjunctiva and lid margin, and is included in the species specified for the antimicrobial preservative effectiveness test. Microorganism contamination of eye drops may occur upon administration of the same via conjunctival sac, that is, the nozzle of the eye drop container contacts with the conjunctiva or lid margin. Accordingly, *Staphylococcus aureus* has the highest possibility to contaminate ophthalmic solution among the species specified for the antimicrobial preservative effectiveness test and therefore, most important strain for evaluating the preservation property of an ophthalmic solution.

In an aqueous composition comprising a 15-keto-PG compound as an active ingredient, effect of a dissolving agent contained in the composition on the antimicrobial properties of a quaternary ammonium type cationic surface active agent such as benzalkonium chloride, especially on the antimicrobial properties against *Staphylococcus aureus* has not been studied well. Nothing has been known about the effect of a castor oil derivative on the antimicrobial properties when added as a dissolving agent to the aqueous composition.
US 2002/0002185 A1 discloses a composition for reducing ocular hypertension which includes: (a) about 0.06 to about 0.24 weight percent isopropyl unoprostone; (b) about 0.3 to about 2 weight percent of two non-ionic surfactant selected from the group consisting of CREMOPHOR RH, BRIJ 97, BRIJ 98, CREMOPHOR EL, Polysorbate 80 and mixtures thereof; (c) about 0.01 to about 0.20 weight percent benzalkonium chloride; (d) about 0.01 to about 0.1 weight percent EDTA; (e) about 0.10 to about 10.0 weight percent mannitol; (f) about 0.01 to about 0.05 molar of an ophthalmically acceptable buffer; (g) an ophthalmically acceptable carrier; in which the pH is adjusted to about 4.5 to about 8.0. A similar disclosure is found in WO 98/41204 A.

### DISCLOSURE OF THE INVENTION

### PROBLEM(S) TO BE SOLVED BY THE INVENTION

An object of the present invention is to provide an aqueous composition for topical administration, especially for topical administration to the eye, comprising a 15-keto-prostaglandin compound as an active ingredient.

### MEANS TO SOLVE THE PROBLEM(S)

Accordingly, the present invention provides an aqueous composition comprising a 15-keto-prostaglandin compound, characterized in that the composition comprises a polyoxyethylene castor oil derivative which is polyoxyethylene 35 castor oil. Preferably, the composition of the invention may further comprise a quaternary ammonium type cationic surface active agent.

### EFFECT(S) OF THE INVENTION

In an aqueous composition comprising a 15-keto-PG compound as an active ingredient, by using a polyoxyethylene castor oil derivative which is a nonionic surfactant in stead of polysorbate, another nonionic surfactant which is conventionally used as a dissolving agent in aqueous compositions, the solubility of 15-ketoprostaglandin compound in water is improved. The aqueous composition of the present invention further comprising a quaternary ammonium type cationic surface active agent will exhibit excellent antimicrobial preservative property against *Staphylococcus aureus* that is the most important species among the species specified in the antimicrobial preservative effectiveness test and therefore, is useful for aqueous ophthalmic composition.

### DETAILED DESCRIPTION OF THE PREFERRED EMBODIMENTS

The nomenclature of the 15-keto-PG compounds used herein is based on the numbering system of prostanoic acid represented in the above formula (A).

The formula (A) shows a basic skeleton of the C-20 PG compound, but the present invention is not limited to those having the same number of carbon atoms. In the formula (A), the numbering of the carbon atoms which constitute the basic skeleton of the PG compounds starts at the carboxylic acid (numbered 1), and carbon atoms in the α-chain are numbered 2 to 7 towards the five-membered ring, those in the ring are 8 to 12, and those in the ω-chain are 13 to 20. When the number of carbon atoms is decreased in the α-chain, the number is deleted in the order starting from position 2; and when the number of carbon atoms is increased in the α-chain, compounds are named as substitution compounds having respective substituents at position 2 in place of the carboxy group (C-1). Similarly, when the number of carbon atoms is decreased in the ω-chain, the number is deleted in the order starting from position 20; and when the number of carbon atoms is increased in the ω-chain, compounds are named as substitution compounds having respective substituents at position 20. Stereochemistry of the compounds is the same as that of the above formula (A) unless otherwise specified.

In general, each of PGD, PGE and PGF represents a PG compound having hydroxy groups at positions 9 and/or 11, but in the present specification they also include those having substituents other than the hydroxy groups at positions 9 and/or 11. Such compounds are referred to as 9-dehydroxy-9-substituted-PG compounds or 11-dehydroxy-11-substituted-PG compounds. A PG compound having hydrogen in place of the hydroxy group is simply named as 9- or 11-dehydroxy compound.

As stated above, the nomenclature of PG compounds is based on the prostanoic acid skeleton. However, in case the compound has a similar partial construction as a prostaglandin, the abbreviation of "PG" may be used. Thus, a PG compound whose α-chain is extended by two carbon atoms, that is, having 9 carbon atoms in the α-chain is named as 2-decarboxy-2-(2-carboxyethyl)-PG compound. Similarly, a PG compound having 11 carbon atoms in the α-chain is named as 2-decarboxy-2-(4-carboxybutyl)-PG compound. Further, a PG compound whose ω-chain is extended by two carbon atoms, that is, having 10 carbon atoms in the ω-chain is named as 20-ethyl-PG compound. These compounds, however, may also be named according to the IUPAC nomenclatures.

The 15-keto-PG compound used in the present invention may be any derivative of a PG insofar as having an oxo group at position 15 in place of the hydroxy group, and may further include a compound having one double bond between positions 13 and 14 (15-keto-PG type 1 compound), two double bonds between positions 13 and 14, and positions 5 and 6 (15-keto-PG type 2 compound), and three double bonds between positions 5 and 6, positions 13 and 14, and positions 17 and 18(15-keto-PG type 3 compound), and a derivative thereof wherein the bond between the positions 13 and 14 is single bond, in place of the double bond (13,14-dihydro-15-keto-PG compound).

Examples of the analogues including substitution compounds or derivatives of the 15-keto-PG compound include a PG compound whose carboxy group at the end of the alpha chain is esterified; physiologically acceptable salt thereof; an unsaturated derivative having a double bond between positions 2 and 3 or a triple bond between positions 5 and 6; PG compounds having substituent(s) on carbon atom(s) at position(s) 3, 5, 6, 16, 17, 18, 19 and/or 20; and PG compounds having lower alkyl or a hydroxy (lower) alkyl group at position 9 and/or 11 in place of the hydroxy group.

According to the present invention, preferred substituents on the carbon atom at position (s) 3, 17, 18 and/or 19 include alkyl having 1-6 carbon atoms, especially methyl and ethyl. Preferred substituents on the carbon atom at position 16 include lower alkyl such as methyl and ethyl, hydroxy, halogen atom such as chlorine and fluorine, and aryloxy such as trifluoromethylphenoxy. Preferred substituents on the carbon atom at position 17 include halogen atom such as chlorine and fluorine. Preferred substituents on the carbon atom at position 20 include saturated or unsaturated lower alkyl such as C₁₋₄ alkyl, lower alkoxy such as C₁₋₄ alkoxy, and lower alkoxy alkyl such as C₁₋₄ alkoxy-C₁₋₄ alkyl. Preferred substituents on the carbon atom at position 5 include halogen atoms such as chlorine and fluorine. Preferred substituents on the carbon atom at position 6 include an oxo group forming a carbonyl group. Stereochemistry of PGs having hydroxy, lower alkyl or hydroxy(lower)alkyl substituent on the carbon atom at positions 9 and 11 may be α, β or a mixture thereof.

Further, the above described derivatives may have a ω chain shorter than that of the primary PGs and a substituent such as alkoxy, cyclohexyl, cyclohexyloxy, phenoxy and phenyl at the end of the truncated ω-chain.

Especially preferred compounds include a 13,14-dihydro-15-keto-PG compound that has a single bond between positions 13 and 14; a 15-keto-20-lower alkyl PG compound that has a lower alkyl, especially ethyl, on the carbon atom at position 20; a 15-keto-PGF compound that has hydroxy groups at positions 9 and 11 of the five memberd ring; a 15-keto-PGE compound that has oxo group at position 9 and hydroxy group at position 11 of the five membered ring; a 11-dehydroxy-15-keto-PGE compound that has oxo group at position 9 and hydrogen atom at position 11 of the five membered ring; and a 15-keto-18,19,20-trinol-17-phenyl-PG compound that has ω-chain shorter than the primary PG compound by three(3) carbon atoms and phenyl group at the end of the ω-chain.

A preferred prostaglandin compound used in the present invention is represented by the formula (I):

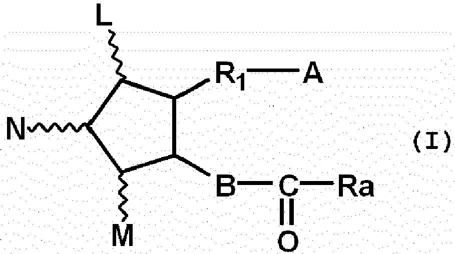

wherein L, M and N are hydrogen, hydroxy, halogen, lower alkyl, hydroxy(lower)alkyl or oxo and the five-membered ring may have at least one double bond;
A is -CH₃, -CH₂OH, -COCH₂OH, -COOH or a functional derivative thereof;
B is -CH₂-CH₂-, -CH=CH- or -C=C-;
R₁ is a saturated or unsaturated bivalent lower or medium aliphatic hydrocarbon residue, which is unsubstituted or substituted with halogen, lower alkyl, hydroxy, oxo, aryl or heterocyclic group, and at least one carbon atom in the aliphatic hydrocarbon is optionally substituted by oxygen, nitrogen or sulfur; and
Ra is a saturated or unsaturated lower or medium aliphatic hydrocarbon group, which is unsubstituted or substituted with halogen, oxo, hydroxy, lower alkyl, lower alkoxy, lower alkanoyloxy, cyclo(lower)alkyl, cyclo(lower)alkyloxy, aryl, aryloxy, heterocyclic group or hetrocyclic-oxy group; lower alkoxy; lower alkanoyloxy; cyclo(lower)alkyl; cyclo(lower)alkyloxy; aryl; aryloxy; heterocyclic group; heterocyclic-oxy group.

A more preferred prostaglandin compound used in the present invention is represented by the formula (II):

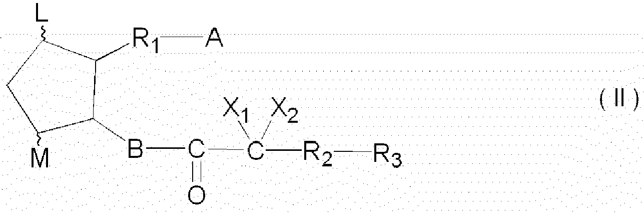

wherein L and M are hydrogen, hydroxy, halogen, lower alkyl, hydroxy(lower)alkyl, lower alkanoyloxy or oxo, and the five-membered ring may have at least one double bond;
A is -CH₃, -CH₂OH, -COCH₂OH, -COOH or a functional derivative thereof;
B is -CH₂-CH₂-, -CH=CH- or -C=C-;
X₁ and X₂ are hydrogen, lower alkyl, or halogen;
R₁ is a saturated or unsaturated bivalent lower or medium aliphatic hydrocarbon residue, which is unsubstituted or substituted with halogen, lower alkyl, hydroxy, oxo, aryl or heterocyclic group, and at least one carbon atom in the aliphatic hydrocarbon is optionally substituted by oxygen, nitrogen or sulfur;
R₂ is a single bond or lower alkylene; and
R₃ is lower alkyl, lower alkoxy, lower alkanoyloxy, cyclo(lower)alkyl, cyclo(lower)alkyloxy, aryl, aryloxy, heterocyclic group or heterocyclic-oxy group.

In the above formula, the term "unsaturated" in the definitions for R₁ and Ra is intended to include at least one or more double bonds and/or triple bonds that are isolatedly, separately or serially present between carbon atoms of the main and/or side chains. According to the usual nomenclature, an unsaturated bond between two serial positions is represented by denoting the lower number of the two positions, and an unsaturated bond between two distal positions is represented by denoting both of the positions.

The term "lower or medium aliphatic hydrocarbon" refers to a straight or branched chain hydrocarbon group having 1 to 14 carbon atoms (for a side chain, 1 to 3 carbon atoms are preferable) and preferably 1 to 10, especially 6 to 10 carbon atoms for R₁ and 1 to 10, especially 1 to 8 carbon atoms for Ra.

The term "halogen atom" covers fluorine, chlorine, bromine and iodine.

The term "lower" throughout the specification is intended to include a group having 1 to 6 carbon atoms unless otherwise specified.

The term "lower alkyl" refers to a straight or branched chain saturated hydrocarbon group containing 1 to 6 carbon atoms and includes, for example, methyl, ethyl, propyl, isopropyl, butyl, isobutyl, sec-butyl, t-butyl, pentyl and hexyl.

The term "lower alkylene" refers to a straight or branched chain bivalent saturated hydrocarbon group containing 1 to 6 carbon atoms and includes, for example, methylene, ethylene, propylene, isopropylene, butylene, isobutylene, t-butylene, pentylene and hexylene.

The term "lower alkoxy" refers to a group of lower alkyl-O-, wherein lower alkyl is as defined above.

The term "hydroxy(lower)alkyl" refers to a lower alkyl as defined above which is substituted with at least one hydroxy group such as hydroxymethyl, 1-hydroxyethyl, 2-hydroxyethyl and 1-methyl-1-hydroxyethyl.

The term "lower alkanoyloxy" refers to a group represented by the formula RCO-O-, wherein RCO- is an acyl group formed by oxidation of a lower alkyl group as defined above, such as acetyl.

The term "cyclo(lower)alkyl" refers to a cyclic group formed by cyclization of a lower alkyl group as defined above but contains three or more carbon atoms, and includes, for example, cyclopropyl, cyclobutyl, cyclopentyl and cyclohexyl.

The term "cyclo(lower)alkyloxy" refers to the group of cyclo(lower)alkyl-O-, wherein cyclo(lower)alkyl is as defined above.

The term "aryl" may include unsubstituted or substituted aromatic hydrocarbon rings (preferably monocyclic groups), for example, phenyl, tolyl, xylyl. Examples of the substituents are halogen atom and halo(lower)alkyl, wherein halogen atom and lower alkyl are as defined above.

The term "aryloxy" refers to a group represented by the formula ArO-, wherein Ar is aryl as defined above.

The term "heterocyclic group" may include mono-to tri-cyclic, preferably monocyclic heterocyclic group which is 5 to 14, preferably 5 to 10 membered ring having optionally substituted carbon atom and 1 to 4, preferably 1 to 3 of 1 or 2 type of hetero atoms selected from nitrogen atom, oxygen atom and sulfur atom. Examples of the heterocyclic group include furyl, thienyl, pyrrolyl, oxazolyl, isoxazolyl, thiazolyl, isothiazolyl, imidazolyl, pyrazolyl, furazanyl, pyranyl, pyridyl, pyridazinyl, pyrimidyl, pyrazinyl, 2-pyrrolinyl, pyrrolidinyl, 2-imidazolinyl, imidazolidinyl, 2-pyrazolinyl, pyrazolidinyl, piperidino, piperazinyl, morpholino, indolyl, benzothienyl, quinolyl, isoquinolyl, purinyl, quinazolinyl, carbazolyl, acridinyl, phenanthridinyl, benzimidazolyl, benzimidazolinyl, benzothiazolyl, phenothiazinyl. Examples of the substituent in this case include halogen, and halogen substituted lower alkyl group, wherein halogen atom and lower alkyl group are as described above.

The term "heterocyclic-oxy group" means a group represented by the formula HcO-, wherein Hc is a heterocyclic group as described above.

The term "functional derivative" of A includes salts, preferably pharmaceutically acceptable salts, ethers, esters and amides.

Suitable "pharmaceutically acceptable salts" include salts formed with non-toxic bases conventionally used in pharmaceutical field, for example a salt with an inorganic base such as an alkali metal salt (such as sodium salt and potassium salt), an alkaline earth metal salt (such as calcium salt and magnesium salt), an ammonium salt; or a salt with an organic base, for example, an amine salt including such as methylamine salt, dimethylamine salt, cyclohexylamine salt, benzylamine salt, piperidine salt, ethylenediamine salt, ethanolamine salt, diethanolamine salt, triethanolamine salt, tris(hydroxymethylamino)ethane salt, monomethyl- monoethanolamine salt, procaine salt and caffeine salt), a basic amino acid salt (such as arginine salt and lysine salt), tetraalkyl ammonium salt and the like. These salts may be prepared by a conventional process, for example from the corresponding acid and base or by salt interchange.

Examples of the ethers include alkyl ethers, for example, lower alkyl ethers such as methyl ether, ethyl ether, propyl ether, isopropyl ether, butyl ether, isobutyl ether, sec-butyl ether, t-butyl ether, pentyl ether and 1-cyclopropyl ethyl ether; and medium or higher alkyl ethers such as octyl ether, diethylhexyl ether, lauryl ether and cetyl ether; unsaturated ethers such as oleyl ether and linolenyl ether; lower alkenyl ethers such as vinyl ether, allyl ether; lower alkynyl ethers such as ethynyl ether and propynyl ether; hydroxy(lower)alkyl ethers such as hydroxyethyl ether and hydroxyisopropyl ether; lower alkoxy (lower)alkyl ethers such as methoxymethyl ether and 1-methoxyethyl ether; optionally substituted aryl ethers such as phenyl ether, tosyl ether, t-butylphenyl ether, salicyl ether, 3,4-di-methoxyphenyl ether and benzamidophenyl ether; and aryl(lower)alkyl ethers such as benzyl ether, trityl ether and benzhydryl ether.

Examples of the esters include aliphatic esters, for example, lower alkyl esters such as methyl ester, ethyl ester, propyl ester, isopropyl ester, butyl ester, isobutyl ester, sec-butyl ester, t-butyl ester, pentyl ester and 1-cyclopropylethyl ester; lower alkenyl esters such as vinyl ester and allyl ester; lower alkynyl esters such as ethynyl ester and propynyl ester; hydroxy(lower)alkyl ester such as hydroxyethyl ester; lower alkoxy (lower) alkyl esters such as methoxymethyl ester and 1-methoxyethyl ester; and optionally substituted aryl esters such as, for example, phenyl ester, tolyl ester, t-butylphenyl ester, salicyl ester, 3,4-di-methoxyphenyl ester and benzamidophenyl ester; and aryl(lower)alkyl ester such as benzyl ester, trityl ester and benzhydryl ester.

The amide of A means a group represented by the formula -CONR'R", wherein each of R' and R" is hydrogen, lower alkyl, aryl, alkyl- or aryl-sulfonyl, lower alkenyl and lower alkynyl, and include for example lower alkyl amides such as methylamide, ethylamide, dimethylamide and diethylamide; arylamides such as anilide and toluidide; and alkyl- or aryl-sulfonylamides such as methylsulfonylamide, ethylsulfonyl-amide and tolylsulfonylamide.

Preferred examples of L and M are a combination wherein both of them are hydroxy which provides a 5-membered ring structure of, so called, PGF type; a combination wherein L is hydroxy and M is oxo which provides a 5-membered ring structure of, so called, PGE type; and a combination wherein L is oxo and M is hydrogen which provides a 5-membered ring structure of, so called, 11-dehydroxy-PG type.

Preferred example of A is -COOH, its pharmaceutically acceptable salt, ester or amide thereof.

Preferred example of B is -CH₂-CH₂- which provides a compound having a structure called as 13,14-dihydro type prostaglandin.

Preferred example of X₁ and X₂ are hydrogen or halogen, more preferably, both are halogen, especially fluorine atoms, which provides a compound having a structure called as 16,16-difluoro type prostaglandin.

Preferred R₁ is a hydrocarbon residue containing 1-10 carbon atoms, preferably 6-10 carbon atoms. Further, at least one carbon atom in the aliphatic hydrocarbon is optionally substituted by oxygen, nitrogen or sulfur.

Examples of R₁ include, for example, the following groups:
- CH₂-CH₂-CH₂-CH₂-CH₂-CH₂-,
- CH₂-CH=CH-CH₂-CH₂-CH₂-,
- CH₂-CH₂-CH₂-CH₂-CH=CH-,
- CH₂ -C≡C-CH₂ -CH₂ -CH₂ -,
- CH₂-CH₂-CH₂-CH₂-CH(CH₃)-CH₂-,
- CH₂-CH₂-CH₂-CH₂-O-CH₂-,
- CH₂-CH=CH-CH₂-O-CH₂-,
- CH₂ -C≡C-CH₂ -O-CH₂ -,
- CH₂-CH₂-CH₂-CH₂-CH₂-CH₂-CH₂-,
- CH₂-CH=CH-CH₂-CH₂-CH₂-CH₂-,
- CH₂-CH₂-CH₂-CH₂-CH₂-CH=CH-,
- CH₂ -C≡C-CH₂ -CH₂ -CH₂ -CH₂ -,
- CH₂-CH₂-CH₂-CH₂-CH₂-CH(CH₃)-CH₂-,
- CH₂-CH₂-CH₂-CH₂-CH₂-CH₂-CH₂-CH₂-,
- CH₂-CH=CH-CH₂-CH₂-CH₂-CH₂-CH₂-,
- CH₂-CH₂-CH₂-CH₂-CH₂-CH₂-CH=CH-,
- CH₂-C≡C-CH₂-CH₂-CH₂-CH₂-CH₂-, and
- CH₂-CH₂-CH₂-CH₂-CH₂-CH₂-CH(CH₃)-CH₂-.

Preferred Ra is a hydrocarbon containing 1-10 carbon atoms, more preferably, 1-8 carbon atoms and most preferably, 5-7 carbon atoms.

The configuration of the ring and the α- and/or ω chains in the above formula (I) and (II) may be the same as or different from that of the primary PGs. However, the present invention also includes a mixture of a compound having a primary type configuration and a compound of a non-primary type configuration.

Typical examples of the compounds used in the present invention are 13,14-dihydro-15-keto-20-ethyl-PGF compound, 13,14-dihydro-15-keto-16,16-difluoro-PGE compound, 11-dehydroxy-13,14-dihydro-15-keto-16,16-difluoro PGE compound, 13,14-dihydro-15-keto-18,19,20-trinol-17-phenyl PGF compound and a derivative or analog thereof.

In the present invention, the 15-keto-PG compound may be in the keto-acetal equilibrium by formation of a hemiacetal between hydroxy at position 11 and keto at position 15.

For example, it has been revealed that when both of X₁ and X₂ are halogen atoms, especially, fluorine atoms, the compound contains a tautomeric isomer, bicyclic compound.

If such tautomeric isomers as above are present, the proportion of both tautomeric isomers varies with the structure of the rest of the molecule or the kind of the substituent present. Sometimes one isomer may predominantly be present in comparison with the other. The 15-keto-PG compound of the present invention includes both isomers.

Further, the 15-keto-PG compounds used in the invention include the bicyclic compound and analogs or derivatives thereof. The bicyclic compound is represented by the formula (III):

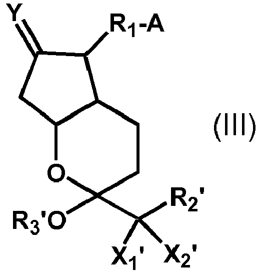

wherein, A is -CH₃, -CH₂OH, -COCH₂OH -COOH or a functional derivative thereof;
X₁' and X₂' are hydrogen, lower alkyl, or halogen;
Y is 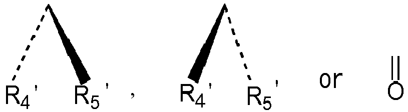 wherein R₄' and R₅' are hydrogen, hydroxy, halogen, lower alkyl, lower alkoxy or hydroxy(lower)alkyl, wherein R₄' and R₅' are not hydroxy and lower alkoxy at the same time.
R₁ is a saturated or unsaturated bivalent lower or medium aliphatic hydrocarbon residue, which is unsubstituted or substituted with halogen, lower alkyl, hydroxy, oxo, aryl or heterocyclic group, and at least one carbon atom in the aliphatic hydrocarbon is optionally substituted by oxygen, nitrogen or sulfur; and
R₂' is a saturated or unsaturated lower or medium aliphatic hydrocarbon residue, which is unsubstituted or substituted with halogen, oxo, hydroxy, lower alkyl, lower alkoxy, lower alkanoyloxy, cyclo(lower)alkyl, cyclo (lower) alkyloxy, aryl, aryloxy, heterocyclic group or hetrocyclic-oxy group; lower alkoxy; lower alkanoyloxy; cyclo(lower)alkyl; cyclo(lower)alkyloxy; aryl; aryloxy; heterocyclic group; heterocyclic-oxy group.
R₃' is hydrogen, lower alkyl, cyclo(lower)alkyl, aryl or heterocyclic group.

Furthermore, while the compounds used in the invention may be represented by a formula or name based on keto-type compound regardless of the presence or absence of the isomers, it is to be noted that such structure or name does not intend to exclude the acetal type compound.

In the present invention, any of isomers such as the individual tautomeric isomers, the mixture thereof, or optical isomers, the mixture thereof, a racemic mixture, and other steric isomers may be used in the same purpose.

Some of the compounds used in the present invention may be prepared by the method disclosed in USP Nos.5,073,569, 5,166,174, 5,221,763, 5,212,324, 5,739,161 and 6,242,485, the contents of these references are herein incorporated by reference.

It has been known that 13,14-dihydro-15-keto-prostaglandin compound having the formula as shown below (Tautomer I) may be in equilibrium with its tautomeric isomer (tautomer II) (See USP 5,166,174, USP 5,225,439, USP 5,284,858, USP 5,380,709, USP 5,428,062 and US 5,886,034, the contents of these references are herein incorporated by reference.)

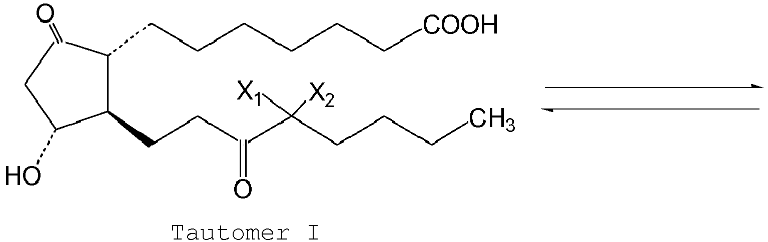

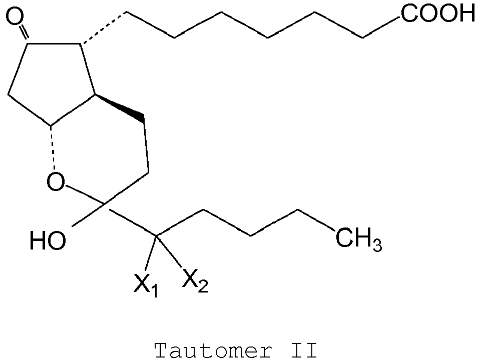

The 15-keto-PG compound described as above is useful for manufacturing pharmaceutical products for various uses and especially useful for the treatment of symptoms in the ophthalmic area such as glaucoma and ocular hypertension.

The term "treatment" or "treating" used herein refers to any means of control of a condition including prevention, cure, relief of the condition, and arrestation or relief of development of the condition.

In the aqueous composition of the present invention, the 15-keto-PG compound, the active ingredient, may be any of the above described compounds.

The concentration of the 15-keto-PG compound in the aqueous composition may vary depending on the specific compound being used, species, age and body weight of the subject to be treated, condition to be treated, desired therapeutic effect, administration amount and treating period and the art can determine a suitable concentration. Typically the dose of the 15-keto-PG compound to provide sufficient effect by systemic administration in accordance with divided dose into one to four fractions per day or under sustained condition may be 0.00001-100mg/kg per day.

According to the present invention, "aqueous composition" refers to a pharmaceutical dosage form which is clear aqueous solution and may be used as ophthalmic drop, nasal drop, ear drop, inhalant, spray, oral administerable product or injectable (intravenous, intraarterial, subcutaneous, intramuscular, intraperitoneal and intraocular) product. The concentration of 15-keto-PG compound in the aqueous composition may generally be about 0.0001 to 10 w/v%, preferably, about 0.0001 to 5 w/v% and more preferably about 0.001 to 1 w/v% based on the total volume of the composition.

The polyoxyethylene castor oil derivative used in the instant invention is a compound obtained by addition polymerization of castor oil or hydrogenated castor oil and ethyleneoxide. According to the invention, polyoxyethylene 35 castor oil (average oxyethylene number: about 35) is used as the polyoxyethylene castor oil derivative.

The amount of the polyoxyethynele castor oil derivative in the aqueous composition of the invention may be about 0.005-20 w/v% and preferably, about 0.01-10 w/v% based on the total volume of the composition.

Examples of the quaternary ammonium type cationic surface active agent used in the invention may include benzalkonium chloride and benzethonium chloride, and benzalkonium chloride is preferably used.

The amount of the quaternary ammonium type cationic surface active agent in the aqueous composition of the invention may be about 0.001-0.05 w/v%, preferably, about 0.001-0.02 w/v% based on the total volume of the composition.

The aqueous composition of the present invention may comprise sole active ingredient or a combination of two or more active ingredients. In case two or more active ingredients are contained in the composition, the amount of each ingredient may be determined in view of the effect and safety of the ingredient.

In addition, the aqueous composition of the present invention may further comprise any other active ingredient in so far as it does not act adverse to the purpose of the present invention.

The aqueous composition of the present invention may further comprise additives that are generally added to conventional aqueous compositions, for example isotonic agent such as sodium chloride, polyols such as glycerine and mannitol, stabilizing agent such as ethylenediamine tetraacetic acid (EDTA), pH buffers such as boric acid, phosphoric acid, acetic acid and salts thereof, in addition to the active ingredient, the polyoxyethylene castor oil derivative (dissolving agent) and a quaternary ammonium type cationic surface active agent.

In the aqueous composition of the present invention, the addition of a polyoxyethylene castor oil derivative as a dissolving agent improves solubility of 15-keto-PG compound in water and therefore, the amount of the dissolving agent in the composition can be decreased. In addition, in an aqueous composition comprising a 15-keto-PG compound as an active ingredient, the addition of a polyoxyethylene castor oil derivative in combination with a quaternary ammonium type cationic surface active agent such as benzalkonium chloride improves the antimicrobial effect of the quaternary ammonium type cationic surface active agent. The aqueous composition of the present invention exhibits excellent antimicrobial preservative property against *Staphylococcus aureus* which is a potential contaminant of ophthalmic aqueous compositions such as eye drops.
The present invention provides, in particular, the following embodiments:
1. An aqueous composition comprising a 15-keto-prostaglandin compound, characterized in that the composition comprises a polyoxyethylene castor oil derivative which is polyoxyethylene 35 castor oil.
2. The aqueous composition of embodiment 1, wherein the 15-keto-prostaglandin compound is a compound of the formula (I): 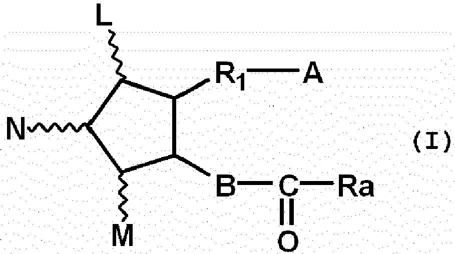 wherein L, M and N are hydrogen, hydroxy, halogen, lower alkyl, hydroxy (lower) alkyl or oxo, and the five-membered ring may have at least one double bond;
   A is -CH₃, -CH₂OH, -COCH₂OH, -COOH or a functional derivative thereof;
   B is -CH₂-CH₂-, -CH=CH- or -C=C-;
   R₁ is a saturated or unsaturated bivalent lower or medium aliphatic hydrocarbon residue, which is unsubstituted or substituted with halogen, lower alkyl, hydroxy, oxo, aryl or heterocyclic group, and at least one carbon atom in the aliphatic hydrocarbon is optionally substituted by oxygen, nitrogen or sulfur; and
   Ra is a saturated or unsaturated lower or medium aliphatic hydrocarbon group, which is unsubstituted or substituted with halogen, oxo, hydroxy, lower alkyl, lower alkoxy, lower alkanoyloxy, cyclo(lower)alkyl, cyclo(lower)alkyloxy, aryl, aryloxy, heterocyclic group or hetrocyclic-oxy group; lower alkoxy; lower alkanoyloxy; cyclo(lower)alkyl; cyclo(lower)alkyloxy; aryl; aryloxy; heterocyclic group; heterocyclic-oxy group.
3. The aqueous composition of embodiment 1, wherein the 15-keto-prostaglandin compound is a 15-keto-20-alkyl-prostaglandin compound.
4. The aqueous composition of embodiment 3, wherein the 15-keto-20-alkyl-prostaglandin compound is a 15-keto-20-ethyl-prostaglandin compound.
5. The aqueous composition of embodiment 1, wherein the 15-keto-prostaglandin compound is a 15-keto-16,16-dihalogen-prostaglandin compound.
6. The aqueous composition of embodiment 5, wherein the 15-keto-16,16-dihalogen-prostaglandin compound is a 15-keto-16,16-difluoro-prostaglandin compound.
7. The aqueous composition of embodiment 1, wherein the 15-keto-prostaglandin compound is a 15-keto-prostaglandin E compound or a 15-keto-prostaglandin F compound.
8. The aqueous composition of embodiment 7, wherein the 15-keto-prostaglandin F compound is a 15-keto-20-ethyl-prostaglandin F_{2α} compound.
9. The aqueous composition of embodiment 7, wherein the 15-keto-prostaglandin E compound is a 15-keto-prostaglandin E₁ compound.
10. The aqueous composition of embodiment 7, wherein the 15-keto-prostaglandin E compound is an 11-dehydroxy-15-keto-prostaglandin E compound.
11. The aqueous composition of embodiment 1, wherein the 15-keto-prostaglandin compound is a 13,14-dihydro-15-keto-prostaglandin compound.
12. The aqueous composition of embodiment 1, wherein the 13,14-dihydro-15-keto-prostaglandin compound is 13,14-dihydro-15-keto-20-ethyl-prostaglandin F_{2α} isopropyl ester.
13. The aqueous composition of embodiment 1, which further comprises a quaternary ammonium type cationic surface active agent.
14. The aqueous composition of embodiment 13, wherein the quaternary ammonium type cationic surface active agent is benzalkonium chloride or benzethonium chloride.
15. The aqueous composition of embodiment 1 or 13 which is for topical administration.
16. The aqueous composition of embodiment 15, which is for ocular topical administration.

The present invention will be explained in more detail by means of the following examples, which are illustrated by way of example only and never intended to limit the scope of the present invention.

### EXAMPLE 1 (for comparison)

Test solution 1 was obtained by dissolving the ingredients in an amount shown below (w/v%) in purified water and sterilized by filter sterilization.

Isopropyl unoprostone, i.e. 13,14-dihydro-15-keto-20-ethyl-PGF2α was used.

| | |
|---|---|
| 0.12% | isopropylunoprostone |
| 0.5% | polyoxyethylene 40 hydrogenated castor oil |
| 0.6% | sodium chloride |
| 0.01% | benzalkonium chloride (BAC) |

Thus obtained test solution was filled in a sterilized container under steric condition and *Staphylococcus aureus* was inoculated to the container and mixed uniformly. The container was kept at 20-25°C and the viable cell count was determined at 6 and 24 hours after the inoculation. The viable cell count determination was conducted by means of the agar plate diffusion test. The log reduction value of the cell number from the inoculated number of the cells over time were calculated. Result is shown in Table 1.

### EXAMPLE 2 (for comparison)

Test solution 2 containing the ingredients shown below was obtained in the same manner as Example 1 with the exception that the amount of benzalkonium chloride was adjusted to 0.015w/v%.

| | |
|---|---|
| 0.12% | isopropylunoprostone |
| 0.5% | polyoxyethylene 40 hydrogenated castor oil |
| 0.6% | sodium chloride |
| 0.015% | benzalkonium chloride |

The antimicrobial preservative effectiveness of the test solution was determined according to the same manner as Example 1. Result is shown in Table 1.

### EXAMPLE 3 (for comparison)

Test solution 3 containing the ingredients shown below was obtained in the same manner as Example 1 with the exception that the amount of benzalkonium chloride was adjusted to 0.02w/v%.

| | |
|---|---|
| 0.12% | isopropylunoprostone |
| 0.5% | polyoxyethylene 40 hydrogenated castor oil |
| 0.6% | sodium chloride |
| 0.02% | benzalkonium chloride |

The antimicrobial preservative effectiveness of the test solution was determined according to the same manner as Example 1. Result is shown in Table 1.

### EXAMPLE 4 (for comparison)

Test solution 4 containing the ingredients shown below was obtained in the same manner as Example 1 with the exception that 0.5w/v% of polyoxyethylene 60 hydrogenated castor oil was used.

| | |
|---|---|
| 0.12% | isopropylunoprostone |
| 0.5% | polyoxyethylene 60 hydrogenated castor oil |
| 0.6% | sodium chloride |
| 0.01% | benzalkonium chloride |

The antimicrobial preservative effectiveness of the test solution was determined according to the same manner as Example 1. Result is shown in Table 1.

### EXAMPLE 5 (for comparison)

Test solution 5 containing the ingredients shown below was obtained in the same manner as Example 2 with the exception that 0.5w/v% of polyoxyethylene 60 hydrogenated castor oil was used.

| | |
|---|---|
| 0.12% | isopropylunoprostone |
| 0.5% | polyoxyethylene 60 hydrogenated castor oil |
| 0.6% | sodium chloride |
| 0.015% | benzalkonium chloride |

The antimicrobial preservative effectiveness of the test solution was determined according to the same manner as Example 1. Result is shown in Table 1.

### EXAMPLE 6 (for comparison)

Test solution 6 containing the ingredients shown below was obtained in the same manner as Example 3 with the exception that 0.5w/v% of polyoxyethylene 60 hydrogenated castor oil was used.

| | |
|---|---|
| 0.12% | isopropylunoprostone |
| 0.5% | polyoxyethylene 60 hydrogenated castor oil |
| 0.6% | sodium chloride |
| 0.02% | benzalkonium chloride |

The antimicrobial preservative effectiveness of the test solution was determined according to the same manner as Example 1. Result is shown in Table 1.

### COMPARATIVE EXAMPLE 1

Test solution 7 containing the ingredients shown below was obtained in the same manner as Example 1 with the exception that 1w/v% of polysorbate 80 was used.

| | |
|---|---|
| 0.12% | isopropylunoprostone |
| 1% | polysorbate 80 |
| 0.6% | sodium chloride |
| 0.01% | benzalkonium chloride |

The antimicrobial preservative effectiveness of the test solution was determined according to the same manner as Example 1. Result is shown in Table 1.

### COMPARATIVE EXAMPLE 2

Test solution 8 containing the ingredients shown below was obtained in the same manner as Example 2 with the exception that 1w/v% of polysorbate 80 was used.

| | |
|---|---|
| 0.12% | isopropylunoprostone |
| 1% | polysorbate 80 |
| 0.6% | sodium chloride |
| 0.015% | benzalkonium chloride |

The antimicrobial preservative effectiveness of the test solution was determined according to the same manner as Example 1. Result is shown in Table 1.

### COMPARATIVE EXAMPLE 3

Test solution 7 was obtained in the same manner as Example 3 with the exception that 1w/v% of polysorbate 80 was used.

| | |
|---|---|
| 0.12% | isopropylunoprostone |
| 1% | polysorbate 80 |
| 0.6% | sodium chloride |
| 0.02% | benzalkonium chloride |

The antimicrobial preservative effectiveness of the test solution was determined according to the same manner as Example 1. Result is shown in Table 1.

**[Table 1]**

| Preservative Effectiveness of the Solution | | | |
|---|---|---|---|
| Test Solution | BAC (%) | Change of Cell Number | |
| | | 6 hours | 24 hours |
| 1 | 0.01 | ND | ND |
| 2 | 0.015 | ND | ND |
| 3 | 0.02 | ND | ND |
| 4 | 0.01 | ND | ND |
| 5 | 0.015 | ND | ND |
| 6 | 0.02 | ND | ND |
| 7 | 0.01 | 0.1 LRV | 3.3 LRV |
| 8 | 0.015 | 0.9 LRV | ND |
| 9 | 0.02 | 1.4 LRV | ND |

| | | | |
|---|---|---|---|
| ND: less than detection limit(< 10 cfu/mL, > 4 LRV) LRV: Log Reduction Value | | | |

According to the above results, the use of polyethylene hydrogenated castor oil as a dissolving agent in an aqueous formulation can reduce the amount of the dissolving agent in the formula than the use of polysorbate 80, another nonionic surface active agent, as the dissolving agent. In addition, the formulations comprising polyethylene hydrogenated castor oil exhibited excellent preservation effectiveness, i.e. the viable cell number after 6 hour was less than the detection limit irrespective of the amount of benzalkonium in the formulation.

### EXAMPLE 7 (for comparison)

Test solution 10 was obtained by dissolving the ingredients in an amount shown below (w/v %) in purified water.

**Compound A (15-keto-18,19,20-trinol-phenyl-PGF_{2α})**

| | |
|---|---|
| 0.2% | compound A |
| 0.5% | polyoxyethylene hydrogenated castor oil |
| 4.6% | mannitol |
| 0.1% | EDTA |
| 0.12% | tromethamine |
| 0.3% | boric acid |
| 0.015% | benzalkonium chloride |

The mixture was stirred overnight and centrifuged to give clear and colorless supernatant. The concentration of compound A in the supernatant was measured by means of liquid chromatography.

The concentration was determined as follows (HPLC method)
Exactly 0.3mL of the supernatant of the test solution 10 was obtained and added with exactly 0.6mL of the internal standard solution and then, added with acetonitrile (HPLC grade) to give exactly 3mL of sample solution. About 0.01g of the reference standard of compound A was weighted precisely and added with acetonitrile (HPLC grade) to give exactly 50mL solution. Exactly 2.5ml of the solution was obtained and added with exactly 1mL of the internal standard solution, and then added with acetonitrile (HPLC grade) to give exactly 5mL of reference solution. Then, 20µl of the sample and reference solution was loaded respectively on the column and analyzed under the condition shown below. The concentration of compound A was calculated by the internal standard method.

HPLC analysis condition:
Detector: ultraviolet light absorptiometer (at 210nm)
Column: approx. 6mm in inner diameter X approx. 15cm in length, stainless steel column packed with octadecylsilane treated silica gel for HPLC (5µm)
Column temperature: 40°C
Mobile phase: mixture of acetonitrile (HPLC grade) and distilled water (HPLC grade)

### EXAMPLE 8 (for comparison)

Test solution 11 containing the ingredients shown below was obtained in the same manner as Example 7 with the exception that the amount of polyoxyethylene hydrogenated castor oil was adjusted to 1.0w/v%.

| | |
|---|---|
| 0.2% | compound A |
| 1.0% | polyoxyethylene hydrogenated castor oil |
| 4.6% | mannitol |
| 0.1% | EDTA |
| 0.12% | tromethamine |
| 0.3% | boric acid |
| 0.015% | benzalkonium chloride |

The concentration of compound A in this solution was determined according to the same manner as Example 7.

### COMPARATIVE EXAMPLE 4

Test solution 12 containing the ingredients shown below was obtained in the same manner as Example 7 with the exception that 0.5w/v% of polysorbate 80 was used.

| | |
|---|---|
| 0.3% | compound A |
| 0.5% | polysorbate 80 |
| 0.77% | sodium chloride |
| 0.1% | EDTA |
| 0.02% | benzalkonium chloride |

The concentration of compound A in this solution was determined according to the same manner as Example 7.

### COMPARATIVE EXAMPLE 5

Test solution 13 containing the ingredients shown below was obtained in the same manner as Example 7 with the exception that 1.0w/v% of polysorbate 80 was used.

| | |
|---|---|
| 0.3% | compound A |
| 1.0% | polysorbate 80 |
| 0.77% | sodium chloride |
| 0.1% | EDTA |
| 0.02% | benzalkonium chloride |

The concentration of compound A in this solution was determined according to the same manner as Example 7. Results are shown in Table 2 below.

**[Table 2]**

| concentration of compound A in test solutions | | |
|---|---|---|
| Test Solution No. | dissolving agent (w/v%) | concentration of compound A (w/v%) |
| 10 | 0.5 | 0.14 |
| 11 | 1.0 | >0.2 |
| 12 | 0.5 | 0.09 |
| 13 | 1.0 | 0.18 |

According to the above results, the solubility of the 15-keto PG in water was higher in a formulation comprising polyoxyethylene hydrogenated castor oil, a nonionic surface active agent, than in a formulation comprising polysorbate 80, another nonionic surface active agent.

## Claims

1. An aqueous composition comprising a 15-keto-prostaglandin compound, **characterized in that** the composition comprises a polyoxyethylene castor oil derivative which is polyoxyethylene 35 castor oil.

2. The aqueous composition of claim 1, wherein the concentration of the 15-keto-prostaglandin compound in the aqueous composition is 0.0001 to 10 w/v% based on the total volume of the composition.

3. The aqueous composition of claim 2, wherein the concentration of the 15-keto-prostaglandin compound in the aqueous composition is 0.0001 to 5 w/v% based on the total volume of the composition.

4. The aqueous composition of claim 3, wherein the concentration of the 15-keto-prostaglandin compound in the aqueous composition is 0.001 to 1 w/v% based on the total volume of the composition.

5. The aqueous composition of claim 1, wherein the amount of the polyoxyethylene castor oil derivative in the aqueous composition is 0.005-20 w/v% based on the total volume of the composition.

6. The aqueous composition of claim 5, wherein the amount of the polyoxyethylene castor oil derivative in the aqueous composition is 0.01-10 w/v% based on the total volume of the composition.

7. The aqueous composition of claim 1, further comprising a quaternary ammonium type cationic surface active agent.

8. The aqueous composition of claim 7, wherein the quaternary ammonium type cationic surface active agent is benzalkonium chloride or benzethonium chloride.

9. The aqueous composition of claim 7, wherein the amount of the quaternary ammonium type cationic surface active agent in the aqueous composition is 0.001-0.05 w/v% based on the total volume of the composition.

10. The aqueous composition of claim 9, wherein the amount of the quaternary ammonium type cationic surface active agent in the aqueous composition is 0.001-0.02 w/v% based on the total volume of the composition.
